# EUROPEAN PATENT APPLICATION

(11) **EP 1 710 308 A1**
(43) Date of publication of application: **11.10.2006**
(21) Application number: 04820305.3
(22) Date of filing: 10.12.2004
(51) Int. Cl.: C12N 15/13, C07K 16/46, C12P 21/02, C12P 21/08

(54) **METHOD OF REINFORCING ANTIBODY ACTIVITY**

(30) Priority: 12.12.2003 JP 2003415760
(71) Applicant: CHUGAI SEIYAKU KABUSHIKI KAISHA, Tokyo, 115-8543 (JP)
(72) Inventor: OHTOMO, Toshihiko, CHUGAI SEIYAKU KABUSHIKI KAISHA, Niihari-gun, Ibaraki3 004101 (JP); YABUTA, Naohiro, CHUGAI SEIYAKU KABUSHIKI KAISHA, Gotenba-shi, Shizuoka 4120038 (JP); TSUNODA, Hiroyuki, CHUGAI SEIYAKU KABUSHIKI KAISHA, Gotenba-shi, Shizuoka 412-0038 (JP); TSUCHIYA, Masayuki CHUGAI SEIYAKU KABUSHIKI KAISHA, Gotenba-shi, Shizuoka 4120038 (JP)
(74) Representative: Bassil, Nicholas Charles
(86) International application number: PCT/JP2004/018493
(87) International publication number: WO 2005/056798

(57) **Abstract**

Anti-human Mpl antibodies were isolated and purified, and then single-chain anti-human Mpl antibodies were prepared using genetic engineering techniques. The antibodies were found to exhibit a high agonistic activity. This shows that the activity of an antibody can be enhanced by making the antibody into a single-chain polypeptide that comprises two or more heavy chain variable regions and two or more light chain variable regions linked via linkers.

## Description

### Technical Field

The present invention relates to methods for enhancing antibody activity.

### Background Art

Antibodies receive attention as pharmaceuticals due to their high stability and low antigenicity in blood. Of these, agonist antibodies which are capable of recognizing cell surface-expressed proteins such as receptors, and thereby induce specific reactions in cells are considered to be useful as pharmaceuticals. Several agonist antibodies such as agonist antibodies against erythropoietin receptor (see Non-patent Document 1), thrombopoietin receptor or CD47 (see Patent Documents 1 and 2) have been reported.

The agonistic activities of these agonist antibodies have been determined by various assay methods; however, the activities are all weaker compared with natural ligands. For example, in order for an agonist antibody to exert its agonistic activity against the thrombopoietin receptor, which belongs to the cytokine receptor family, it is essential that the TPO receptor is first dimerized and placed at a distance appropriate for signal transduction. Since the antibody molecules are divalent, they can easily dimerize the receptor. However, since the antibodies are large molecules with a molecular weight of approximately 150kD, they have a low structural flexibility. Expectantly, it would be difficult for bound receptor to provide the optimal distance for signal transduction and to exhibit a sufficient agonistic activity.
Patent Document 1: WO 02/33072
Patent Document 2: WO 02/33073
Non-patent Document 1: Elliott S et al., J. Biol. Chem., 1996, Vol. 271(40), p. 24691-24697

### Disclosure of the Invention

The present invention was achieved in view of the circumstances described above. An objective of the present invention is to provide methods for enhancing antibody activity. Specifically, the objective is to provide methods for enhancing antibody activity, which comprise preparing a single-chain polypeptide in which two or more light chain variable regions are linked to two or more heavy chain variable regions via linkers.

A minibody, specifically, a diabody or an sc(Fv)2 that has a molecular weight of about 60 kD, which is less than half of a full-size antibody, is predicted to have a relatively high degree of structural flexibility, and is thought to dimerize a receptor more efficiently or as efficiently as a ligand, thereby exhibiting higher activity.

The present inventors prepared and purified an anti-human Mpl antibody, and then constructed a single-chain antibody from the anti-human Mpl antibody VB22B using genetic engineering techniques. Further, the inventors constructed an expression vector for the anti-human Mpl antibody sc(Fv)2, and transiently expressed the single-chain antibody in CHO-DG44 cells. Then, the inventors obtained the single-chain anti-human Mpl antibody VB22B sc(Fv)2 from the culture supernatant. As control, an expression vector for an anti-human Mpl diabody was constructed and expressed in COS7 cells, and the VB22B diabody was obtained from the culture supernatant. Both antibodies were evaluated for their TPO-like agonist activities, and the single-chain antibody was found to exhibit a higher agonistic activity. This finding shows that the activity of an antibody can be enhanced by converting the antibody to a single-chain polypeptide comprising two or more heavy chain variable regions and two or more light chain variable regions linked via linkers.

Specifically, the present invention relates to methods for enhancing antibody activity, more specifically to:
[1] a method for enhancing the activity of an antibody, which comprises making the antibody into a single-chain polypeptide comprising two or more light chain variable regions and two or more heavy chain variable regions linked via linkers;
[2] a method for enhancing the activity of an antibody, which comprises linking a first polypeptide to a second polypeptide by a linker, wherein the first polypeptide comprises the antibody's heavy chain variable region and light chain variable region and the second polypeptide comprises the antibody's heavy chain variable region and light chain variable region;
[3] a method for enhancing the activity of an antibody, which comprises converting the antibody into an sc(Fv)2;
[4] the method according to any one of [1] to [3], wherein the activity is an agonistic activity;
[5] the method according to any one [1] to [4], wherein the linker is a peptide linker;
[6] the method according to [5], wherein the length of the peptide linker is 5 to 30 amino acids;
[7] the method according to [6], wherein the length of the peptide linker is 12 to 18 amino acids;
[8] the method according to [7], wherein the length of the peptide linker is 15 amino acids;
[9] an antibody whose activity has been enhanced by the method according to any one of [1] to [8];
[10] a method for producing the antibody of [9], which comprises:
   (a) preparing a DNA that encodes two or more antibody heavy chain variable regions, two or more antibody light chain variable regions, and peptide linkers linking each of the variable regions;
   (b) constructing a vector comprising the DNA,
   (c) introducing the vector into a host cell, and
   (d) culturing the host cell;
[11] the production method according to [10], wherein the DNA encodes two heavy chain variable regions, two light chain variable regions, and three peptide linkers; and
[12] the production method according to [11], wherein the DNA is encoded in the order of: heavy chain variable region, peptide linker, light chain variable region, peptide linker, heavy chain variable region, peptide linker, and light chain variable region.

### Brief Description of the Drawings

Fig. 1 shows the amino acid sequence of an anti-human Mpl antibody (H and L chains). The H chain amino acid sequences of VB140, VB45B, VB22B, VB16, and TA136 are shown as SEQ ID NOs: 19, 20, 21, 22, and 23, respectively. Further, the L chain amino acid sequences of VB140, VB45B, VB22B, VB16, and TA136 are shown in SEQ ID NOs: 24, 25, 26, 27, and 28 respectively.
Fig. 2 shows the process for constructing a single-chain antibody, sc(Fv)2.
Fig. 3 illustrates the result of evaluating VB22B antibody's agonistic activity using BaF3-human Mpl.
Fig. 4 illustrates the result of evaluating VB22B antibody's agonistic activity using BaF3-monkey Mpl.
Fig. 5 illustrates the result of evaluating VB16 antibody's agonistic activity using BaF3-human Mpl.
Fig. 6 illustrates the result of evaluating VB 140 antibody's agonistic activity using BaF3-human Mpl.
Fig. 7 illustrates the result of evaluating VB45B antibody's agonistic activity using BaF3-human Mpl.
Fig. 8 illustrates the result of evaluating TA136 antibody's agonistic activity using BaF3-human Mpl.

### Best Mode for Carrying Out the Invention

The present invention provides methods for enhancing antibody activity, which comprise converting the antibody into a single-chain polypeptide comprising two or more light chain variable regions and two or more heavy chain variable regions linked via linkers. The antibodies of the present invention, whose activity is to be enhanced, may be any kinds of antibodies including antibodies derived from any kinds of animals such as mouse, human, rat, rabbit, and camel. Furthermore, the antibodies may be any antibodies including, for example, altered antibodies that contain amino acid substitutions, such as chimeric antibodies and humanized antibodies, as well as antibodies modified by linking with various molecules, antibody fragments, and antibodies with modified sugar chains.

In the present invention, an antibody whose activity is enhanced may be a whole antibody or minibody such as diabody.

The single-chain polypeptides of the present invention include, for example, single-chain polypeptides in which a first polypeptide comprising antibody heavy chain and light chain variable regions and a second polypeptide comprising antibody heavy chain and light chain variable regions are linked via linkers.

The first polypeptide comprising antibody heavy chain and light chain variable regions may be the same as or different from the second polypeptide comprising heavy chain and light chain variable regions. If the first and second polypeptides are different, the antibody may be an antibody that recognizes one antigen or epitope, or a bispecific antibody recognizing different antigens or epitopes.

An exemplary polypeptide comprising antibody heavy chain and light chain variable regions is scFv (single-chain Fv). Thus, sc(Fv)2 is an example of a single-chain polypeptide, in which a first polypeptide comprising antibody heavy chain and light chain variable regions and a second polypeptide comprising antibody heavy chain and light chain variable regions are linked via linkers. sc(Fv)2 is a single-chain polypeptide antibody that comprises two heavy chain variable regions and two light chain variable regions linked via linkers (Hudson et al, J Immunol. Methods 1999 ; 231:177-189).

For sc(Fv)2, the two heavy chain variable regions (VH) and the two light chain variable regions (VL) can be linked in any order without particular limitation. Examples of the arrangement are listed below.
[VH]- linker- [VL] -linker - [VI-1] - linker - [VL]
[VL] - linker - [VH] -linker- [VH] - linker - [VL]
[VH] -linker- [VL] -linker - [VL] - linker - [VH]
[VH] -linker- [VH] -linker - [VL] - linker- [VL]
[VL] -linker - [VL] -linker- [VH] - linker- [VH]
[VL] -linker - [VH] -linker- [VL] - linker - [VH]

In the present invention, sc (Fv) 2 is preferably arranged in the order of: [VH] linker [VL] linker [VH] linker [VL].

The amino acid sequences of the heavy chain variable regions or light chain variable regions may contain substitutions, deletions, additions and/or insertions. Furthermore, the antibodies may also lack portions of the heavy chain variable regions and/or light chain variable regions, and an alternative polypeptide(s) may be added thereto, as long as they have antigen-binding ability. In addition, the variable regions may be chimerized or humanized.

Alterations of amino acid sequences, such as amino acid substitutions, deletions, additions, and/or insertions, humanization and chimerization, can be achieved after the activity is enhanced by the methods of the present invention. Alternatively, activity enhancement by the methods of the present invention may be done after amino acid sequences are altered.

Chimeric antibodies are antibodies prepared by combining sequences derived from different animal species, and include for example, antibodies comprising the heavy chain and light chain variable regions of a murine antibody, and the heavy chain and light chain constant regions of a human antibody. Chimeric antibodies can be prepared by known methods. For example, a DNA encoding the V region of an antibody is linked to a DNA encoding the C region of a human antibody, and the construct is inserted into an expression vector and introduced into a host to produce chimeric antibodies.

Humanized antibodies are also referred to as "reshaped human antibodies". Such a humanized antibody is obtained by transferring the complementarity-determining region (CDR) of an antibody derived from a non-human mammal, for example mouse, to the complementarity-determining region of a human antibody, and the general gene recombination procedure for this is also known (see European Patent Application No. 125023 and WO 96/02576).

Specifically, a DNA sequence designed to link a murine antibody CDR to the framework region (FR) of a human antibody can be synthesized by PCR, using primers prepared from several oligonucleotides containing overlapping portions of both CDR and FR terminal regions (see methods described in WO 98/13388).

The human antibody framework region to be linked by CDR is selected in order to form a favorable antigen-binding site in the complementarity-determining region. Amino acids of the framework region in the antibody variable region may be substituted, as necessary, for the complementarity-determining region of the reshaped human antibody to form a suitable antigen-binding site (Sato, K. et al., 1993, Cancer Res. 53, 851-856).

The constant region of a human antibody is used as the C region of a chimeric antibody or humanized antibody. For example, Cγ1, Cγ2, Cγ3, and Cγ4 can be used as the H chain, and Cκ and Cλ can be used as the L chain. The human antibody C region may be modified to improve the antibody or the stability of the antibody production.

Generally, chimeric antibodies comprise the variable region of an antibody from a non-human mammal and the constant region derived from a human antibody. On the other hand, humanized antibodies comprise the complementarity-determining region of an antibody from a non-human mammal, and the framework region and C region derived from a human antibody.

In addition, amino acids of the variable region (for example, FR) and the constant region of a prepared chimeric antibody or humanized antibody may be substituted with other amino acids. The sequence of the antibody variable region may be a sequence of a variable region of any known antibody, or an antibody sequence prepared using an appropriate antigen by methods known to those skilled in the art. Specifically, the sequence can be determined by, for example, the following procedure. Immunized animals such as mice are immunized with an antigen by a conventional immunization method. The immunocytes obtained are fused with known parental cells by a conventional cell fusion method and screened for monoclonal antibody-producing cells (hybridomas) by a conventional screening method. The antigen can be prepared by known methods. Hybridomas can be prepared, for example, by the method of Milstein *et al.* (Kohler, G. and Milstein, C., Methods Enzymol. (1981) 73:3-46). If the antigen has a low immunogenicity, it may be linked with an immunogenic macromolecule such as albumin, and then used for immunization. Then, the antibody variable region (V region) cDNA is synthesized from the hybridoma mRNA using reverse transcriptase, and the obtained cDNA sequence is determined by known methods.

Meanwhile, methods for preparing human antibodies are also well known. Desired human antibodies with binding activity can be obtained by, for example, sensitizing human lymphocytes in vitro and fusing the sensitized lymphocytes with human myeloma cells that are capable of permanent cell division (see Japanese Patent Application Kokoku Publication No. (JP-B) H1-59878 (examined, approved Japanese patent application published for opposition)). Alternatively, an antigen is administered to a transgenic animal having a repertoire of all human antibody genes, and antibody-producing cells are produced and immortalized to obtain human antibodies against the antigen (see International Patent Application WO 94/25585, WO 93/12227, WO92/03918, and WO 94/02602).

In the present invention, arbitrary peptide linkers that can be introduced by genetic engineering, or synthetic linkers (for example, linkers disclosed in "Protein Engineering, 9(3), 299-305, 1996"), can be used as linkers to link a heavy chain variable region and a light chain variable region.

There are no limitations as to the length of the peptide linkers. The length can be appropriately selected by those skilled in the art according to the purpose, and is typically 1 to 100 amino acids, preferably 5 to 30 amino acids, and more preferably 12 to 18 amino acids (e.g., 15 amino acids).

Amino acid sequences of such peptide linkers include, for example:
Ser
Gly·Ser
Gly·Gly·Ser
Ser·Gly·Gly
Gly·Gly·Gly·Ser
Ser·Gly·Gly·Gly
Gly·Gly·Gly·Gly·Ser
Ser·Gly·Gly·Gly·Gly
Gly·Gly·Gly·Gly·Gly·Ser
Ser·Gly·Gly·Gly·Gly·Gly
Gly·Gly·Gly·Gly·Gly·Gly·Ser
Ser·Gly·Gly·Gly·Gly·Gly·Gly
(Gly·Gly·Gly·Gly·Ser)n
(Ser·Gly·Gly·Gly·Gly)n
where n is an integer of 1 or larger.

Synthetic linkers (chemical crosslinking agents) include crosslinking agents that are routinely used to crosslink peptides, for example, N-hydroxy succinimide (NHS), disuccinimidyl suberate (DSS), bis(sulfosuccinimidyl) suberate (BS³), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl] sulfone (BSOCOES), and bis[2-(sulfosuccinimidoxycarbonyloxy)ethyl] sulfone (sulfo-BSOCOES). These crosslinking agents are commercially available..

The present invention also provides antibodies whose activities have been improved by methods described above.

The present invention also provides methods for producing antibodies, which comprise:
(a) preparing a DNA encoding two or more antibody heavy chain variable regions, two or more antibody light chain variable regions, and peptide linkers to be used for linking each of the variable regions;
(b) preparing a vector comprising the DNA;
(c) introducing the vector into a host cell; and
(d) culturing the host cell.

In these methods, first, a DNA that encodes two or more antibody heavy chain variable regions, two or more antibody light chain variable regions, and peptide linkers that link the variable regions is prepared. Such DNA includes, for example, DNA encoding two heavy chain variable region (VH) and two light chain variable region (VL), and three peptide linkers. A preferred DNA is an sc (Fv)2-encoding DNA.

The two VHs and two VLs can be linked in any order without particular limitation. Examples of the arrangement are listed below.
[VH] -linker- [VL]- linker- [VH] -linker- [VL]
[VL] -linker - [VH] -linker- [VH] -linker - [VL]
[VH] - linker- [VL] - linker- [VL] - linker v[VH]
[VH] -linker- [VH] -linker- [VL] -linker - [VL]
[VL] -linker- [VL] -linker- [VH] -linker- [VH]
[VL] -linker- [VH] - linker- [VL] - linker- [VH]
In the present invention, a preferred arrangement is: [VH] - linker- [VL] -linker- [VH] -linker- [VL].

The amino acid sequences of the heavy chain variable regions or light chain variable regions may contain substitutions, deletions, additions and/or insertions. Furthermore, the antibody may also lack portions of the heavy chain variable regions or/and light chain variable regions, as long as it has an antigen-binding activity. In addition, the variable regions may be chimerized or humanized.

The present invention further includes construction of vectors containing the DNAs mentioned above.

When *E. coli* is used as a host, there is no particular limitation as to the type of vector of the present invention, as long as the vector contains an "ori" responsible for its replication in *E*. *coli* and a marker gene. The "ori" ensures the amplification and mass production of the vector in *E. coli* (for example, JM109, DH5α, HB101, and XL1Blue). The marker gene is used to select the *E*. *coli* transformants (for example, a drug resistance gene selected by an appropriate drug such as ampicillin, tetracycline, kanamycin, and chloramphenicol). The vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs.

In particular, expression vectors are useful as vectors of the present invention. When an expression vector is expressed, for example, in *E. coli,* it should have the above characteristics in order to be amplified in *E. coli*. Additionally, when *E. coli,* such as JM109, DH5α, HB101, or XL1-Blue are used as the host cell, the vector preferably has a promoter, for example, lacZ promoter (Ward et al. (1989) Nature 341:544-546; (1992) FASEB J. 6:2422-2427), araB promoter (Better et al. (1988) Science 240:1041-1043), or T7 promoter, that allows efficient expression of the desired gene in *E. coli.* Other examples of the vectors include pGEX-5X-1 (Pharmacia), "QIAexpress system" (QIAGEN), pEGFP, and pET (where BL21, a strain expressing T7 RNA polymerase, is preferably used as the host).

Furthermore, the vectors may comprise a signal sequence for polypeptide secretion. When producing polypeptides into the periplasm of *E. coli,* the pelB signal sequence (Lei, S. P. *et al.* J. Bacteriol. 169:4379 (1987)) may be used as a signal sequence for polypeptide secretion. For example, calcium chloride methods or electroporation methods may be used to introduce the vector into a host cell.

In addition to *E*. *coli,* expression vectors derived from mammals (e.g., pCDNA3 (Invitrogen), pEGF-BOS (Nucleic Acids Res. (1990) 18(17):5322), pEF, pCDM8), insect cells (e.g., "Bac-to-BAC baculovirus expression system" (GIBCO-BRL), pBacPAK8), plants (e.g., pMH1, pMH2), animal viruses (e.g., pHSV, pMV, pAdexLcw), retroviruses (e.g., pZIPneo), yeasts (e.g., "Pichia Expression Kit" (Invitrogen), pNV11, SP-Q01), and *Bacillus subtilis* (e.g., pPL608, pKTH50) may also be used as a vector of the present invention.

In order to express proteins in animal cells such as CHO, COS, and NIH3T3 cells, the vector preferably has a promoter necessary for expression in such cells, for example, an SV40 promoter (Mulligan et al. (1979) Nature 277:108), MMLV-LTR promoter, EF1α promoter (Mizushima et al. (1990) Nucleic Acids Res. 18:5322), CMV promoter, etc. It is even more preferable that the vector also carries a marker gene for selecting transformants (for example, a drug-resistance gene selected by a drug such as neomycin and G418). Examples of vectors with such characteristics include pMAM, pDR2, pBK-RSV, pBK-CMV, pOPRSV, and pOP13, and such.

In addition, to stably express a gene and amplify the gene copy number in cells, CHO cells that are defective in the nucleic acid synthesis pathway are introduced with a vector containing a DHFR gene (for example, pCHOI) to compensate for the defect, and the copy number is amplified using methotrexate (MTX). Alternatively, a COS cell, which carries an SV40 T antigen-expressing gene on its chromosome, can be transformed with a vector containing the SV40 replication origin (for example, pcD) for transient gene expression. The replication origin may be derived from polyoma virus, adenovirus, bovine papilloma virus (BPV), and such. Furthermore, to increase the gene copy number in host cells, the expression vector may contain, as a selection marker, aminoglycoside transferase (APH) gene, thymidine kinase (TK) gene, *E*. *coli* xanthine guanine phosphoribosyl transferase (Ecogpt) gene, dihydrofolate reductase (dhfr) gene, and such.

In the present invention, next, the vector is introduced into a host cell. The host cells into which the vector is introduced are not particularly limited, for example, *E*. *coli* and various animal cells are available for this purpose. The host cells may be used, for example, as a production system to produce and express polypeptides comprising the two or more antibody heavy chain variable regions and the two or more antibody light chain variable regions, and peptide linkers linking each of the variable regions in the present invention. *In vitro* and *in vivo* production systems are available for polypeptide production systems. Production systems that use eukaryotic cells or prokaryotic cells are examples of *in vitro* production systems.

Eukaryotic cells that can be used are, for example, animal cells, plant cells, and fungal cells. Known animal cells include: mammalian cells, for example, CHO (J. Exp. Med. (1995)108, 945), COS, 3T3, myeloma, BHK (baby hamster kidney), HeLa, Vero, amphibian cells such as *Xenopus laevis* oocytes (Valle, et al. (1981) Nature 291, 358-340), or insect cells (e.g., Sf9, Sf21, and Tn5). In the present invention, CHO-DG44, CHO-DXB11, COS7 cells, and BHK can be suitably used. Among animal cells, CHO cells are particularly favorable for large-scale expression. Vectors can be introduced into a host cell by, for example, calcium phosphate methods, the DEAE-dextran methods, methods using cationic liposome DOTAP (Boehringer-Mannheim), electroporation methods, lipofection methods.

Plant cells include, for example, Nicotiana tabacum-derived cells known as a protein production system. Calluses may be cultured from these cells. Known fungal cells include yeast cells, for example, genus Saccharomyces such as Saccharomyces cerevisiae and Saccharomyces pombe; and filamentous fungi, for example, genus Aspergillus such as Aspergillus niger.

Bacterial cells can be used in the prokaryotic production systems. Examples of bacterial cells include *E. coli* (for example, JM109, DH5α, HB101 and such); and Bacillus subtilis.

Next, the above host cells are cultured. Antibodies can be obtained by transforming the cells with a DNA of interest and *in vitro* culturing of these transformants. Transformants can be cultured using known methods. For example, DMEM, MEM, RPMI 1640, or IMDM may be used as the culture medium for animal cells, and may be used with or without serum supplements such as FBS or fetal calf serum (FCS). Serum-free cultures are also acceptable. The preferred pH is about 6 to 8 during the course of culturing. Incubation is carried out typically at a temperature of about 30 to 40°C for about 15 to 200 hours. Medium is exchanged, aerated, or agitated, as necessary.

On the other hand, production systems using animal or plant hosts may be used as systems for producing polypeptides *in vivo.* For example, a DNA of interest is introduced into an animal or plant and the polypeptide is produced in the body of the animal or plant and then recovered. The "hosts" of the present invention includes such animals and plants.

Animals to be used for the production system include mammals or insects. Mammals such as goats, pigs, sheep, mice, and cattle may be used (Vicki Glaser SPECTRUM Biotechnology Applications (1993)). Alternatively, the mammals may be transgenic animals.

For example, a DNA of interest is prepared as a fusion gene with a gene encoding a polypeptide specifically produced in milk, such as the goat β-casein gene. DNA fragments containing the fusion gene are injected into goat embryos, which are then introduced back to female goats. The desired protein can be obtained from milk produced by the transgenic goats, which are born from the goats that received the embryos, or from their offspring. Appropriate hormones may be administered to increase the volume of milk containing the protein produced by the transgenic goats (Ebert, K.M. et al., Bio/Technology 12, 699-702 (1994)).

Insects such as silkworms, may also be used. Baculoviruses carrying a DNA encoding a protein of interest can be used to infect silkworms, and the antibody of interest can be obtained from the body fluids (Susumu, M. et al., Nature 315, 592-594 (1985)).

Plants used in the production system include, for example, tobacco. When tobacco is used, a DNA encoding an antibody of interest is inserted into a plant expression vector, for example, pMON 530, and then the vector is introduced into a bacterium, such as Agrobacterium tumefaciens. The bacteria are then used to infect tobacco such as Nicotiana tabacum, and the desired antibodies can be recovered from the leaves (Julian K.-C. Ma et al., Eur. J. Immunol. 24, 131-138 (1994)).

The resulting antibody may be isolated from the inside or outside (such as the medium) of host cells, and purified as a substantially pure and homogenous antibody. Methods are not limited to any specific method and any standard method for isolating and purifying antibodies may be used. Antibodies may be isolated and purified, by selecting an appropriate combination of, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, recrystallization, and others.

Chromatographies include, for example, affinity chromatographies, ion exchange chromatographies, hydrophobic chromatographies, gel filtrations, reverse-phase chromatographies, and adsorption chromatographies (Strategies for Protein Purification and Characterization: A Laboratory Course Manual. Ed Daniel R. Marshak et al., Cold Spring Harbor Laboratory Press, 1996). These chromatographies can be carried out using liquid phase chromatographies such as HPLC and FPLC. Examples of the affinity chromatography columns include protein A columns and protein G columns. Examples of the proteins A columns include Hyper D, POROS, and Sepharose F. F. (Pharmacia).

An antibody can be modified freely and peptide portions deleted by treating the antibody with an appropriate protein modifying enzyme before or after antibody purification. Such protein modifying enzymes include, for example, trypsins, chymotrypsins, lysyl endopeptidases, protein kinases, and glucosidases.

The antibody activities to be enhanced in the present invention include, but are not limited to, binding activity, neutralizing activity, cell damaging activity, agonistic activity, antagonistic activity, and enzymatic activity. However, the activity is preferably an activity that leads to quantitative and/or qualitative changes or effects in an organism, tissue, cell, protein, DNA, RNA, and such. In particular, agonistic activity is preferred.

The term "agonistic activity" refers to an activity that induces changes in some physiological activities through intracellular signal transduction that results from the binding of an antibody to an antigen such as receptor. The physiological activities include, for example, proliferation activity, survival activity, differentiation activity, transcriptional activity, membrane transport activity, binding activity, proteolytic activity, phosphorylation/dephosphorylation activity, oxidation/reduction activity, transfer activity, nucleolytic activity, dehydration activity, cell death-inducing activity, and apoptosis-inducing activity, without being limited thereto.

The antigens of the present invention are not particularly limited, and can be any kind of antigen. Such antigens include, for example, receptors, cancer antigens, MHC antigens, and differentiation antigens.

The receptors include, for example, receptors that belong to receptor families such as the hematopoietic factor receptor family, cytokine receptor family, tyrosine kinase receptor family, serine/threonine kinase receptor family, TNF receptor family, G protein-coupled receptor family, GPI-anchored receptor family, tyrosine phosphatase receptor family, adhesion factor family, and hormone receptor family. Various references that relate to receptors belonging to these receptor families and their characteristics are available and include, for example, Cooke BA., King RJB., van der Molen HJ. ed. New Comprehensive Biochemistry Vol. 18B "Hormones and their Actions Part II" pp. 1-46 (1988) Elsevier Science Publishers BV., New York, USA; Patthy L. (1990) Cell, 61: 13-14; Ullrich A., et al. (1990) Cell, 61: 203-212; Massagul J. (1992) Cell, 69: 1067-1070; Miyajima A., et al. (1992) Annu. Rev. Immunol., 10: 295-331; Taga T. and Kishimoto T. (1992) FASEB J., 7: 3387-3396; Fantl WI., et al. (1993) Annu. Rev. Biochem., 62: 453-481; Smith CA., et al. (1994) Cell, 76: 959-962; Flower DR. (1999) Biochim. Biophys. Acta, 1422: 207-234; and M. Miyasaka ed., Cell Technology, supplementary volume, Handbook series, "Handbook for Adhesion Factors" (1994) (Shujunsha, Tokyo, Japan).

Specifically, receptors belonging to the above-described receptor families include, for example, the following human and mouse receptors: erythropoietin (EPO) receptors, granulocyte colony-stimulating factor (G-CSF) receptors, thrombopoietin (TPO) receptors, insulin receptors, Flt-3 ligand receptors, platelet-derived growth factor (PDGF) receptors, interferon (IFN)-α and -β receptors, leptin receptors, growth hormone (GH) receptors, interleukin (IL)-10 receptors, insulin-like growth factor (IGF)-I receptors, leukemia inhibitory factor (LIF) receptors, and ciliary neurotrophic factor (CNTF) receptors (hEPOR: Simon, S. et al. (1990) Blood 76, 31-35.; mEPOR: D'Andrea, AD. et al. (1989) Cell 57, 277-285; hG-CSFR: Fukunaga, R. et al. (1990) Proc. Natl. Acad. Sci. USA. 87, 8702-8706; mG-CSFR: Fukunaga, R. et al. (1990) Cell 61, 341-350; hTPOR: Vigon, I. et al. (1992) 89, 5640-5644; mTPOR: Skoda, RC. et al. (1993) 12, 2645-2653; hInsR: Ullrich, A. et al. (1985) Nature 313, 756-761; hFlt-3: Small, D. et al. (1994) Proc. Natl. Acad. Sci. USA. 91, 459-463; hPDGFR: Gronwald, RGK. et al. (1988) Proc. Natl. Acad. Sci. USA. 85, 3435-3439; hIFN α/βR: Uze, G. et al. (1990) Cell 60, 225-234; and Novick, D. et al. (1994) Cell 77, 391-400).

Cancer antigens are antigens expressed as a result of malignant cell alteration, and are also referred to as "cancer-specific antigens". Aberrant sugar chains that are presented on cell surface and on protein molecules due to malignant cell transformation may also serve as cancer antigens, and are referred to as, in particular, "cancer-related carbohydrate antigens". Cancer antigens include, for example, CA19-9, CA15-3, and sialyl SSEA-1 (SLX).

MHC antigens are broadly divided into MHC class I and II antigens. Class I MHC antigens include HLA-A, -B, -C, -E, -F, -G, and -H, and Class II MHC antigens include HLA-DR, -DQ, and -DP.

Differentiation antigens include CD1, CD2, CD3, CD4, CD5, CD6, CD7, CD8, CD10, CD11a, CD11b, CD11c, CD 13, CD 14, CD15s, CD16, CD18, CD 19, CD20, CD21, CD23, CD25, CD28, CD29, CD30, CD32, CD33, CD34, CD35, CD38, CD40, CD41a, CD41b, CD42a, CD42b, CD43, CD44, CD45, CD45RO, CD48, CD49a, CD49b, CD49c, CD49d, CD49e, CD49f, CD51, CD54, CD55, CD56, CD57, CD58, CD61, CD62E, CD62L, CD62P, CD64, CD69, CD71, CD73, CD95, CD102, CD106, CD122, CD126, and CDw130.

There is no limitation as to the type of detection indicators to be used for determining activity changes, as long as the indicator can monitor quantitative and/or qualitative changes. For example, it is possible to use cell-free assay indicators, cell-based assay indicators, tissue-based assay indicators, and *in vivo* assay indicators.

Indicators that can be used in cell-free assays include enzymatic reactions, quantitative and/or qualitative changes in proteins, DNAs, or RNAs. Such enzymatic reactions include, for example, amino acid transfers, sugar transfers, dehydrations, dehydrogenations, and substrate cleavages. Alternatively, protein phosphorylations, dephosphorylations, dimerizations, multimerizations, hydrolyses, dissociations and such; DNA or RNA amplifications, cleavages, and extensions can be used as the indicator in cell-free assays. For example, protein phosphorylations downstream of a signal transduction pathway may be used as a detection indicator.

Alterations in cell phenotype, for example, quantitative and/or qualitative alterations in products, alterations in growth activity, alterations in cell number, morphological alterations, or alterations in cellular properties, can be used as the indicator in cell-based assays. The products include, for example, secretory proteins, surface antigens, intracellular proteins, and mRNAs. The morphological alterations include, for example, alterations in dendrite formation and/or dendrite number, alteration in cell flatness, alteration in cell elongation/axial ratio, alterations in cell size, alterations in intracellular structure, heterogeneity/homogeneity of cell populations, and alterations in cell density. Such morphological alterations can be observed under a microscope. Cellular properties to be used as the indicator include anchor dependency, cytokine-dependent response, hormone dependency, drug resistance, cell motility, cell migration activity, pulsatory activity, and alteration in intracellular substances. Cell motility includes cell infiltration activity and cell migration activity. The alterations in intracellular substances include, for example, alterations in enzyme activity, mRNA levels, levels of intracellular signaling molecules such as Ca²⁺ and cAMP, and intracellular protein levels. When a cell membrane receptor is used, alterations in the cell proliferating activity induced by receptor stimulation can be used as the indicator.

The indicators to be used in tissue-based assays include functional alterations adequate for the subject tissue. In *in vivo* assays, alterations in tissue weight, alterations in the blood system (for example, alterations in blood cell counts, protein contents, or enzyme activities), alterations in electrolyte levels, and alterations in the circulating system (for example, alterations in blood pressure or heart rate).

The methods for measuring such detection indices are not particularly limited. For example, absorbance, luminescence, color development, fluorescence, radioactivity, fluorescence polarization, surface plasmon resonance signal, time-resolved fluorescence, mass, absorption spectrum, light scattering, and fluorescence resonance energy transfer may be used. These measurement methods are known to those skilled in the art and may be selected appropriately depending on the purpose.

For example, absorption spectra can be obtained by using a conventional photometer, plate reader, or such; luminescence can be measured with a luminometer or such; and fluorescence can be measured with a fluorometer or such. Mass can be determined with a mass spectrometer. Radioactivity can be determined with a device such as a gamma counter depending on the type of radiation. Fluorescence polarization can be measured with BEACON (TaKaRa). Surface plasmon resonance signals can be obtained with BIACORE. Time-resolved fluorescence, fluorescence resonance energy transfer, or such can be measured with ARVO or such. Furthermore, a flow cytometer can also be used for measuring. It is possible to use one of the above methods to measure two or more different types of detection indices. A greater number of detection indices may also be examined by using two or more measurement methods simultaneously and/or consecutively. For example, fluorescence and fluorescence resonance energy transfer can be measured at the same time with a fluorometer.

In the present invention, the agonistic activity can be determined by assay methods known to those skilled in the art. For example, the activity can be determined by the measurement methods described in the EXAMPLES, using cell proliferation as an indicator. More specifically, an antibody whose agonistic activity is to be determined is added to cells that proliferate in an agonist-dependent manner, followed by incubation of the cells. Then, a reagent such as WST-8, which undergoes a chromogenic reaction at a specific wavelength depending on the viable cell count, is added and the absorbance is measured. The agonistic activity can be determined using the measured absorbance as an indicator.

Cells that proliferate in an agonist-dependent manner can also be prepared by methods known to those skilled in the art. For example, when the antigen is a receptor capable of transducing cell growth signals, cells expressing the receptor may be used. Alternatively, when the antigen is a receptor that cannot transduce signals, a chimeric receptor comprising the intracellular domain of a receptor that transduces cell growth signals and the extracellular domain of a receptor that does not transduce cell growth signals can be prepared for cellular expression. Receptors that transduce cell growth signals include, for example, G-CSF receptors, mpl, neu, GM-CSF receptors, EPO receptors, c-kit, and FLT-3. Cells that can be used to express a receptor include, for example, BaF3, NFS60, FDCP-1, FDCP-2, CTLL-2, DA-1, and KT-3.

All prior art documents cited herein are incorporated herein by reference.

### Examples

Herein below, the present invention will be specifically described with reference to

### Examples.

### [Example 1] Preparation of anti-human Mpl antibodies

### 1.1 Establishment of Mpl-expressing BaF3 cell lines

BaF3 cell lines expressing the full-length Mpl gene were established to obtain cell lines that proliferate in a TPO-dependent manner. A full-length human Mpl cDNA (Palacios, R. et al., Cell, 41, 727-734 (1985)) (GenBank accession NO. NM_005373) was amplified by PCR. The cDNA was cloned into a pCOS2 expression vector to construct pCOS2-hMplfull. The expression vector pCOS2 was constructed by removing the DHFR gene expression region from pCHOI (Hirata, Y. et al., FEBS Letter, 356, 244-248 (1994)), where the neomycin resistance gene expression region from HEF-VH-gγ1 (Sato, K. et al., Mol Immunol., 31, 371-381 (1994)) is inserted. The cynomolgus monkey Mpl cDNA ((SEQ ID NO: 1) and the amino acid sequence of a protein encoded thereby (SEQ ID NO: 2)) was cloned from total RNA extracted from the bone marrow cells of cynomolgus monkey, using a SMART RACE cDNA Amplification Kit (Clontech). The resulting cynomolgus monkey cDNA was inserted into pCOS2 to construct pCOS2-monkeyMplfull.

Each vector (20 µg) prepared as described above was mixed with BaF3 cells (1x 10⁷ cells/mL) suspended in PBS in Gene Pulser cuvettes. This mixture was then pulsed at 0.33 kV and 950 µFD using a Gene Pulser II (Bio-Rad). The BaF3 cells introduced with the above DNAs by electroporation were added to RPMI 1640 medium (Invitrogen) containing 1 ng/mL mouse interleukin 3 (hereinafter abbreviated as mIL-3; Peprotech), 500 µg/mL Geneticin (Invitrogen), and 10% FBS (Invitrogen), and selected to establish a human Mpl-expressing BaF3 cell line (hereinafter abbreviated as "BaF3-human Mpl"), and a monkey Mpl-expressing BaF3 cell line (hereinafter abbreviated as BaF3-monkey Mpl). Following selection, these cells were cultured and maintained in RPMI 1640 containing 1 ng/mL rhTPO (R&D) and 10% FBS.

### 1.2 Establishment of Mpl-expressing CHO cell lines

CHO cell lines expressing the full-length Mpl gene were established to obtain cell lines to be used for assessing binding activity by flow cytometry. First, the DHFR gene expression site from pCHOI was inserted into pCXN2 (Niwa, H. et al., Gene, 108, 193-199 (1991)) at the HindIII site to prepare a pCXND3expression vector. The respective Mpl genes were amplified by PCR using pCOS2-hMplfull, and pCOS2-monkeyMplfull as templates, and primers with a His-tag sequence. The PCR products were cloned into pCXND3 to construct pCXND3-hMpl-His, and pCXND3-monkey Mpl-His, respectively.

Vectors thus prepared (25 µg each) were mixed with a PBS suspension of CHO-DG44 cells (1x10⁷ cells/mL) in Gene Pulser cuvettes. The mixture was then pulsed at 1.5 kV and 25 µFD using Gene Pulser II (Bio-Rad). The CHO cells introduced with these DNAs by electroporation were added to CHO-S-SFMII medium (Invitrogen) containing 500 µg/mL Geneticin and 1x HT (Invitrogen). A human Mpl-expressing CHO cell line (hereinafter abbreviated as "CHO-human Mpl"), and a monkey Mpl-expressing CHO cell line (hereinafter abbreviated as "CHO-monkey Mpl") were established through selection.

### 1.3 Preparation of soluble human Mpl protein

To prepare soluble human Mpl protein, an expression system using insect Sf9 cells for production and secretion of the protein was constructed as described below. A DNA construct encoding the extracellular region of human Mpl (Gln 26 to Trp 491) with a downstream FLAG tag was prepared. The construct was inserted into a pBACSurf-1 Transfer Plasmid (Novagen) between the PstI and SmaI sites to prepare pBACSurfl-hMpl-FLAG. Then, Sf9 cells were transformed with 4 µg of pBACSurfl-hMpl-FLAG using the Bac-N-Blue Transfection Kit (Invitrogen). The culture supernatant was collected after a three-day incubation. Recombinant virus was isolated by plaque assays. The prepared virus stock was used to infect Sf9 cells, and the culture supernatant was collected.

Soluble human Mpl protein was purified from the obtained culture supernatant as described below. The culture supernatant was loaded onto a Q Sepharose Fast Flow (Amersham Biosciences) for adsorption, and the adsorbed protein was then eluted with 50 mM Na-phosphate buffer (pH7.2) containing 0.01 % (v/v) Tween 20 and 500 mM NaCl. After the eluates were loaded onto a FLAG M2-Agarose (Sigma-Aldrich) for adsorption, the protein adsorbed was eluted with 100 mM glycine-HCl buffer (pH3.5) containing 0.01 % (v/v) Tween 20. Immediately after elution, the fraction obtained was neutralized with 1 M Tris-HCl Buffer (pH8.0) and the buffer was exchanged with PBS(-) and 0.01% (v/v) Tween 20 using PD-10 columns (Amersham Biosciences). The purified soluble Mpl protein was referred to as "shMpl-FLAG".

### 1.4 Preparation of human Mpl-IgG Fc fusion protein

Human fusion protein Mpl-IgG Fc gene was prepared according to the method by Bennett *et al.* (Bennett, B. D. et al., J. Biol. Chem. 266, 23060-23067 (1991)). A nucleotide sequence encoding the extracellular region of human Mpl (Gln 26 to Trp 491) was linked to a nucleotide sequence encoding the Fc region of human IgG-γ1 (a region downstream of Asp 216). A BstEII sequence (amino acids: Val-Thr) was attached to the junction as a fusion linker between these two regions. A 19-amino acid signal peptide derived form human IgG H chain variable region was used as the signal sequence. The resulting human fusion protein Mpl-IgG Fc gene was cloned into pCXND3 to construct pCXND3-hMpl-Fc.

The vectors thus prepared (25 µg) was each mixed with a PBS suspension of CHO-DG44 cells (1 x 10⁷ cells/mL) in Gene Pulser cuvettes. The mixture was then pulsed at 1.5 kV and 25 µFD using Gene Pulser II (Bio-Rad). The CHO cells introduced with the DNA by electroporation were added to CHO-S-SFMII medium containing 500 µg/mL Geneticin and 1x HT (Invitrogen). shMPL-Fc-expressing CHO cell line (CHO-hMpl-Fc) was then established through selection.

Human Mpl-IgG Fc fusion protein was purified from the culture supernatant as described below. The culture supernatant was loaded onto a Q Sepharose Fast Flow (Amersham Biosciences) for adsorption, and then the adsorbed protein were eluted with 50 mM Na-phosphate buffer (pH7.6) containing 0.01 % (v/v) Tween 20 and 1 M NaCl. After the eluates were loaded onto a HiTrap protein G HP column (Amersham Biosciences) for adsorption, the adsorbed protein was eluted with 0.1 M glycine-HCl buffer (pH2.7) containing 150 mM NaCl and 0.01% (v/v) Tween 20. Immediately after elution, the obtained fraction was neutralized with 1 M Tris-HCl Buffer (pH8.0) and the buffer was exchanged with PBS(-) and 0.01% (v/v) Tween 20 using PD-10 columns (Amersham Biosciences). The purified soluble Mpl protein was referred to as "hMpl-Fc".

### 1.5 Immunization with shMpl-FLAG or BaF3-human Mpl and hybridoma selection

MRL/MpJUmmCrj-lpr/lpr mice (hereinafter abbreviated as "MRL/lpr mice"; purchased from Charles River, Japan) were immunized; the primary immunization was carried out at eight weeks of age. For every single mouse, an emulsion containing 100 µg of shMPL-FLAG combined with Freud's complete adjuvant (H37 Ra; Beckton Dickinson), was administered subcutaneously as the primary injection. As a booster injection, an emulsion containing shMPL-FLAG (50 µg per mouse) combined with Freud's incomplete adjuvant (Beckton Dickinson) was administered subcutaneously. Three mice which have been immunized six times in total were subjected to a final injection of shMPL-FLAG (50 µg per mouse) through the caudal vein. Cell fusion was achieved by mixing the mouse myeloma P3-X63Ag8U1 cells (P3U1; purchased from ATCC) and mouse splenocytes using polyethylene glycol 1500 (Roche Diagnostics). Hybridoma selection in HAT medium began the following day and culture supernatants were obtained. Screening was carried out by ELISA, using immunoplates with immobilized shMpl-FLAG or hMpl-Fc and the assayed cell proliferation activity of BaF3-hMpl as an index. Positive clones were isolated as single clones by limiting dilution and then cultured on a large scale. The culture supernatants were collected. Anti-human Mpl antibody-producing hybridomas, VB22B, VB16, VB140, and VB45B, were obtained by this method.

In addition, Balb/C mice (Charles River Laboratories, Japan) were intraperitoneally administered with 1.0 x 10⁷ cells of BaF3-human Mpl for a total of eleven times over a period of one week to five months. Spleen cells from these mice were fused with mouse myeloma cell P3U1 as described above. Cell selection was carried out the next day using a HAT medium and screening was performed using as an index the cell proliferation activity of Baf3-hMpl in the culture supernatant. Positive clones were isolated as single clones by limiting dilution and then cultured on a large scale. The culture supernatants were collected. An anti-human Mpl antibody-producing hybridoma, TA136, was obtained by this method.

### 1.6 Analyses of anti-human Mpl antibodies

Antibody concentrations were determined by carrying out a mouse IgG sandwich ELISA using goat anti-mouse IgG (gamma) (ZYMED) and alkaline phosphatase-goat anti-mouse IgG (gamma) (ZYMED), generating a calibration curve by GraphPad Prism (GraphPad Software; USA), and calculating the antibody concentrations from the calibration curve. Commercially available antibodies of the same isotype were used as standards.

Antibody isotypes were determined by antigen-dependent ELISA using isotype-specific secondary antibodies. hMpl-Fc was diluted to 1 µg/mL with a coating buffer (0.1 mM NaHCO₃, pH9.6) containing 0.02% (w/v) NaN₃, and then added to ELISA plates. The plates were incubated overnight at 4°C for coating. The plates were blocked with a diluent buffer (50 mM Tris-HCl (pH8.1) containing 1 mM MgCl₂, 150 mM NaCl, 0.05% (v/v) Tween 20, 0.02% (w/v) NaN₃, 1% (w/v) BSA). After the addition of hybridoma culture supernatants, the plates were allowed to stand at room temperature for 1 hr. After washing with a rinse buffer (0.05% (v/v) Tween 20 in PBS), alkaline phosphatase-labeled isotype-specific secondary antibodies were added to the plates. Then, the plates were allowed to stand at room temperature for 1 hr. Color development was carried out using SIGMA104 (Sigma-Aldrich) diluted to 1 mg/mL with a substrate buffer (50 mM NaHCO₃, pH9.8) containing 10 mM MgCl₂, and absorbance was measured at 405 nm using Benchmark Plus (Bio-Rad).

The binding activities of an antibody to shMpl-FLAG and hMPL-Fc were determined by ELISA. ELISA plates were coated with 1 µg/mL of purified shMpl-FLAG or hMPL-Fc, and blocked with a diluent buffer. Hybridoma culture supernatants were added to the plates, and the plates were allowed to stand at room temperature for 1 hr. Then, alkaline phosphatase-labeled anti-mouse IgG antibodies (Zymed) were added to the plates. Color development was similarly carried out using the above method. Following a one-hour coloring reaction at room temperature, absorbance was measured at 405 nm and EC₅₀ values were computed using GraphPad Prism.

CHO-human Mpl cells and CHO-monkey Mp1 cells were harvested, and suspended in FACS Buffer (1% FBS/ PBS) to a final concentration of 1x 10⁶ cells/mL. The suspensions were aliquoted into Multiscreen (Millipore) at 100 µl/well, and the culture supernatants were removed by centrifugation. Culture supernatants diluted to 5 µg/mL were added to the plates and incubated on ice for 30 min. The cells were washed once with FACS buffer, and incubated on ice for 30 min following the addition of an FITC-labeled anti-mouse IgG antibody (Beckman Coulter). After incubation, the mixture was centrifuged at 500 rpm for 1 min. The supernatants were removed, and then the cells were suspended in 400 µL of FACS buffer. The samples were analyzed by flow cytometry using EPICS ELITE ESP (Beckman Coulter). An analysis gate was set on the forward and side scatters of a histogram to include viable cell populations.

Agonistic activities of an antibody were evaluated using BaF3-human Mpl and BaF3-monkey Mpl which proliferate in a TPO-dependent manner. Cells of each cell line were suspended at 4 x 10⁵ cells/ml in RPMI 1640/10% FBS (Invitrogen), and each suspension was aliquoted into a 96-well plate at 60µl/well. A 40 µL aliquot of rhTPO (R&D) and hybridoma culture supernatants prepared at various concentrations was added into each well. The plates were then incubated at 37°C under 5% CO₂ for 24 hr. A 10-µL aliquot of the Cell Count Reagent SF (Nacalai Tesque) was added into each well. After incubation for 2 hr, absorbance was measured at 450 nm (and at 655 nm as a control) using a Benchmark Plus. EC₅₀ values were calculated using GraphPad Prism.

The above analysis yielded human Mp1-binding antibodies, VB 22B, VB16, VB140, VB45B, and TA 136.

### 1.7 Purification of anti-human Mpl antibodies

Anti-human Mpl antibodies were purified from hybridoma culture supernatants as described below. After the culture supernatants were loaded onto HiTrap protein G HP columns (Amersham Biosciences) for adsorption, the antibodies were eluted with 0.1 M glycine-HCl (pH2.7) Buffer. Immediately after elution, the fractions were neutralized with 1 M Tris-HCl Buffer (pH9.0), and dialyzed against PBS to replace the buffer for one day.

### [Example 2] Preparation of single-chain anti-human Mpl antibodies

Examples for preparing single-chain antibodies from the VB22B anti-human Mpl antibody are described below.

### 2.1 Cloning of the anti-human Mpl antibody variable region

The variable region was amplified by RT-PCR using total RNA extracted from hybridomas producing anti-human Mpl antibodies. Total RNA was extracted from 1x 10⁷ hybridoma cells using the RNeasy Plant Mini Kit (QIAGEN).

A 5'-terminal fragment of the gene was amplified from 1 µg of total RNA by the SMART RACE cDNA Amplification Kit (Clontech), using a synthetic oligonucleotide MHC-IgG2b (SEQ ID NO: 3) complementary to mouse IgG2b constant region or a synthetic oligonucleotide kappa (SEQ ID NO: 4) complementary to mouse κ chain constant region. Reverse transcription was carried out at 42°C for 1.5 hr.

The composition of the PCR reaction solution (50 µL in total) is shown below.

| | |
|---|---|
| 10x Advantage 2 PCR Buffer (Clontech) | 5 µL |
| 10x Universal Primer A Mix (Clontech) | 5 µL |
| dNTPs (dATP, dGTP, dCTP, and dTTP) (Clontech) | 0.2 mM |
| Advantage 2 Polymerase Mix (Clontech) | 1 µL |
| Reverse transcription product | 2.5 µL |
| Synthetic oligonucleotide, MHC-IgG2b or kappa | 10 pmol |

The PCR reaction conditions were:
94°C (initial temperature) for 30 sec;
five cycles of 94°C for 5 sec and 72°C for 3 min;
five cycles of 94°C for 5 sec, 70°C for 10 sec, and 72°C for 3 min;
25 cycles of 94°C for 5 sec, 68°C for 10 sec, and 72°C for 3 min; and final extension was at 72°C for 7 min.

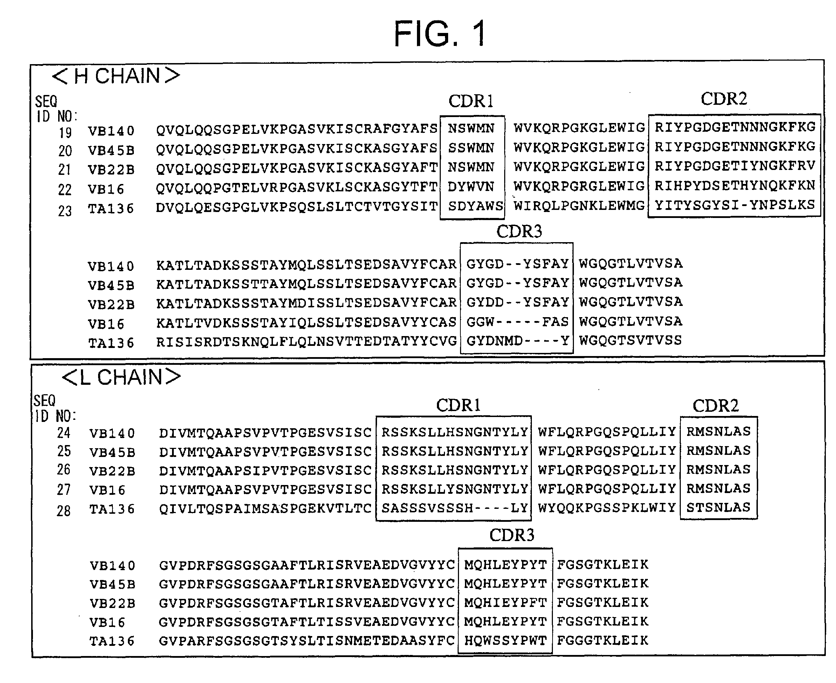

The PCR products were purified from agarose gel using the QIAquick Gel Extraction Kit (QIAGEN), and cloned into a pGEM-T Easy Vector (Promega). The nucleotide sequence was then determined using the ABI 3700 DNA Analyzer (Perkin Elmer). The nucleotide sequence of cloned VB22B H chain variable region (hereinafter abbreviated as "VB22B-VH") is shown in SEQ ID NO: 5, and the amino acid sequence encoded thereby is shown in SEQ ID NO: 6. The nucleotide sequence of the L chain variable region (hereinafter abbreviated as "VB22B-VL") is shown in SEQ ID NO: 7, and the amino acid sequence encoded thereby is shown in SEQ ID NO: 8. The amino acid sequences of VB22B, VB16, VB140, VB45B, and TA136 are shown in Fig. 1.

### 2.2 Preparation of expression vectors for anti-human Mpl diabodies

A gene encoding a VB22B single-chain Fv (hereinafter abbreviated as "VB22B diabody") containing a five-amino acid linker sequence was constructed, by linking a nucleotide sequence encoding a (Gly₄Ser)₁ linker to the VB22B-VH-encoding gene at its 3' end and to the VB22B-VL-encoding gene at its 5' end; both genes had been amplified by PCR.

The VB22B-VH forward primer, 70-115HF, (SEQ ID NO: 9) was designed to contain an EcoRI site. The VB22B-VH reverse primer, 33·115HR, (SEQ ID NO: 10) was designed to hybridize to a DNA encoding the C terminus of VB22B-VH, and to have a nucleotide sequence encoding the (Gly₄Ser)₁ linker and a nucleotide sequence hybridizing to the DNA encoding the N terminus of VB22B-VL. The VB22B-VL forward primer, 33-115LF, (SEQ ID NO: 11) was designed to have a nucleotide sequence encoding the N terminus of VB22B-VL, a nucleotide sequence encoding the (Gly₄Ser)₁ linker, and a nucleotide sequence encoding the C terminus of VB22B-VH. The VB22B-VL reverse primer, 33·115LR, (SEQ ID NO: 12) was designed to hybridize to a DNA encoding the C terminus of VB22B-VL and to have a nucleotide sequence encoding a FLAG tag (Asp Tyr Lys Asp Asp Asp Asp Lys/SEQ ID NO: 13) and a NotI site.

In the first round of PCR, two PCR products: one containing VB22B-VH and a linker sequence, and the other containing VB22B-VL and the identical linker sequence, were synthesized by the procedure described below.

The composition of the PCR reaction solution (50 µL in total) is shown below.

| | |
|---|---|
| 10x PCR Buffer (TaKaRa) | 5 µL |
| dNTPs (dATP, dGTP, dCTP, and dTTP) (TaKaRa) | 0.4 mM |
| DNA polymerase TaKaRa Ex Taq (TaKaRa) | 2.5 units |
| pGEM-T Easy vector comprising VB22B-VH or VB22B-VL gene | 10 ng |
| Synthetic oligonucleotides, 70-115HF and 33-115HR, or 33-115LF and 33-115LR | 10 pmol |

The PCR reaction conditions were:
94°C (initial temperature) for 30 sec;
five cycles of: 94°C for 15 sec and 72°C for 2 min;
five cycles of 94°C for 15 sec and 70°C for 2 min;
28 cycles of 94°C for 15 sec and 68°C for 2 min;
and final extension was at 72°C for 5 min.

After the PCR products of about 400 bp were purified from agarose gel using the QIAquick Gel Extraction Kit (QIAGEN), the second-round PCR was carried out using aliquots of the respective PCR products according to the protocol described below.

The composition of the PCR reaction solution (50 µL in total) is shown below.

| | |
|---|---|
| 10x PCR Buffer (TaKaRa) | 5 µL |
| dNTPs (dATP, dGTP, dCTP, and dTTP) (TaKaRa) | 0.4 mM |
| DNA polymerase TaKaRa Ex Taq (TaKaRa) | 2.5 unit |
| First-round PCR products (two types) | 1 µL |
| Synthetic oligonucleotides, 70-115HF and 33·115LR | 10 pmol |

The reaction conditions were:
94°C (initial temperature) for 30 sec;
five cycles of 94°C for 15 sec and 72°C for 2 min;
five cycles of 94°C for 15 sec and 70°C for 2 min;
28 cycles of 94°C for 15 sec and 68°C for 2 min;
and final extension was at 72°C for 5 min.

The PCR products of about 800 bp were purified from agarose gel using the QIAquick Gel Extraction Kit (QIAGEN), and then digested with EcoRI and NotI (both from TaKaRa). The resulting DNA fragments were purified using the QIAquick PCR Purification Kit (QIAGEN), and then cloned into pCXND3 to prepare pCXND3-VB22B db.

### 2.3 Preparation of expression vectors for anti-human Mpl antibody sc(Fv)2

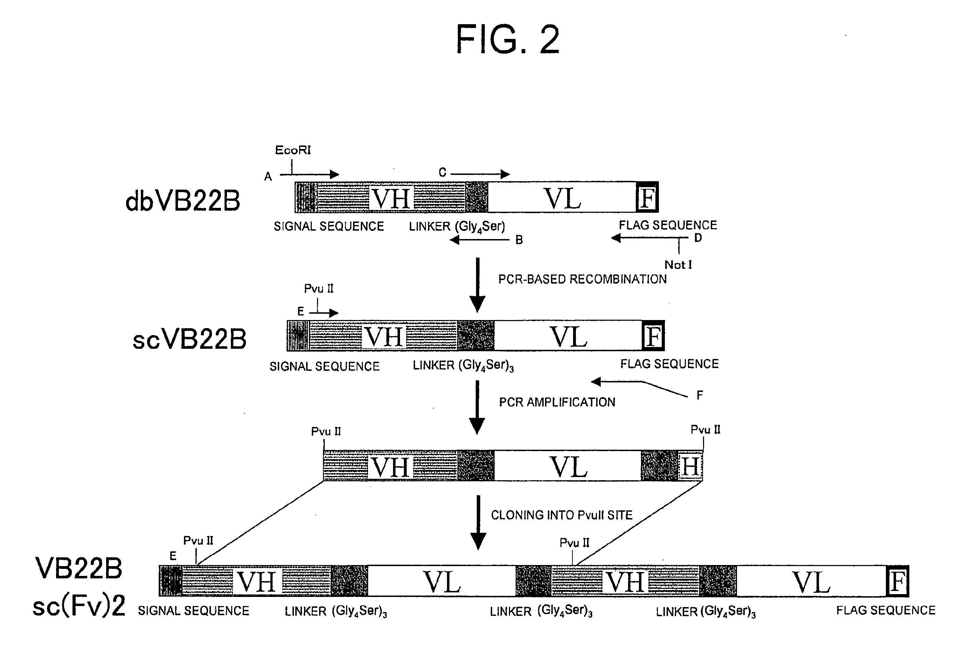

To prepare expression plasmids for the modified antibody [sc(Fv)2] comprising two units of H chain variable region and two units of L chain variable region derived from VB22B, the above-described pCXND3-VB22B db was modified by PCR using the procedure shown below. The process for constructing the sc(Fv)2 gene is illustrated in Fig. 2.

First, PCR method was carried out to amplify (a) the VB22B-VH-encoding gene in which a nucleotide sequence encoding a 15-amino acid linker (Gly₄Ser)₃ was added to its 3' end; and (b) the VB22B-VL-encoding gene containing the identical linker nucleotide sequence added to its 5' end. The desired construct was prepared by linking these amplified genes. Three new primers were designed in this construction process. The VB22B-VH forward primer, VB22B-fpvu, (primer A; SEQ ID NO: 14) was designed to have an EcoRI site at its 5' end and to convert Gln22 and Leu23 of VB22B db into a PvuII site. The VB22B-VH reverse primer, sc-rL15, (primer B; SEQ ID NO: 15) was designed to hybridize to a DNA encoding the C terminus of VB22B-VH, and to have a nucleotide sequence encoding the (Gly₄Ser)₃ linker, as well as a nucleotide sequence hybridizing to a DNA encoding the N terminus of VB22B-VL. The VB22B-VL forward primer, sc-fL15, (primer C; SEQ ID NO: 16) was designed to have a nucleotide sequence encoding the N terminus of VB22B-VL, a nucleotide sequence encoding the (Gly₄Ser)₃ linker, and a nucleotide sequence encoding the C terminus of VB22B-VH.

In the first-round PCR, two PCR products: one comprising VB22B-VH and a linker sequence, and the other comprising VB22B-VL and the identical linker sequence, were synthesized by the procedure described below.

The composition of the PCR reaction solution (50 µL in total) is shown below.

| | |
|---|---|
| 10x PCR Buffer (TaKaRa) | 5 µL |
| dNTPs (dATP, dGTP, dCTP, and dTTP) (TaKaRa) | 0.4 mM |
| DNA polymerase TaKaRa Ex Taq (TaKaRa) | 2.5 units |
| pCXND3-VB22B db | 10 ng |
| Synthetic oligonucleotides, VB22B-fpvu, sc-rL15 or sc-fL15, and 33·115LR (primer D) | 10 pmol |

The reaction conditions were:
94°C (initial temperature) for 30 sec;
five cycles of 94°C for 15 sec and 72°C for 2 min;
five cycles of 94°C for 15 sec and 70°C for 2 min;
28 cycles of 94°C for 15 sec and 68°C for 2 min;
and final extension was at 72°C for 5 min.

After the PCR products of about 400 bp were purified from agarose gel using the QIAquick Gel Extraction Kit (QIAGEN), the second-round PCR was carried out using aliquots of the respective PCR products according to the protocol described below.

The composition of the PCR reaction solution (50 µL in total) is shown below.

| | |
|---|---|
| 10x PCR Buffer (TaKaRa) | 5 µL |
| dNTPs (dATP, dGTP, dCTP, and dTTP) (TaKaRa) | 0.4 mM |
| DNA polymerase TaKaRa Ex Taq (TaKaRa) | 2.5 units |
| First-round PCR product (two types) | 1 µL |
| Synthetic oligonucleotide, 70-115HF and 33-115LR | 10 pmol |

The reaction conditions were:
94°C (initial temperature) for 30 sec;
five cycles of 94°C for 15 sec and 72°C for 2 min;
five cycles of 94°C for 15 sec and 70°C for 2 min;
28 cycles of 94°C for 15 sec and 68°C for 2 min;
and final extension was at 72°C for 5 min.

The PCR products of about 800 bp were purified from agarose gel using the QIAquick Gel Extraction Kit (QIAGEN), and then digested with EcoRI and NotI (both from TaKaRa). The resulting DNA fragments were purified using the QIAquick PCR Purification Kit (QIAGEN), and then cloned into pBacPAK9 (Clontech) to construct pBacPAK9-scVB22B.

A fragment to be inserted into the PvuII site of pBacPAK9-scVB22B was prepared. Specifically, the fragment has a PvuII recognition site at both ends and a nucleotide sequence, in which a gene encoding the VB22B-VH N-terminus is linked, via a (Gly₄Ser)₃ linker-encoding nucleotide sequence, to a gene encoding the amino acid sequence of an N terminus-deleted VB22B-VH linked to VB22B-VL via the (Gly₄Ser)₃ linker. Two primers were newly designed to prepare the fragment by PCR. The forward primer for the fragment of interest, Fv2-f (primer E; SEQ ID NO: 17), was designed to have a PvuII site at its 5' end and a VB22B-VH 5'-end sequence. The reverse primer for the fragment of interest, Fv2-r (primer F; SEQ ID NO: 18), was designed to hybridize to a DNA encoding the C terminus of VB22B-VL, and to have a PvuII site, a nucleotide sequence encoding the (Gly₄Ser)₃ linker, and a nucleotide sequence hybridizing to a DNA encoding the N terminus of VB22B-VH. PCR was carried out using pBacPAK9-scVB22B as a template as described below. The composition of the PCR reaction solution (50 µL in total) is shown below.

| | |
|---|---|
| 10x PCR Buffer (TaKaRa) | 5 µL |
| dNTPs (dATP, dGTP, dCTP, and dTTP) (TaKaRa) | 0.4 mM |
| DNA polymerase TaKaRa Ex Taq (TaKaRa) | 2.5 units |
| pBacPAK9-scVB22B | 10 µg |
| Synthetic oligonucleotide, Fv2-f and Fv2-r | 10 pmol |

The reaction conditions were:
94°C (initial temperature) for 30 sec;
five cycles of 94°C for 15 sec and 72°C for 2 min;
five cycles of 94°C for 15 sec and 70°C for 2 min;
28 cycles of 94°C for 15 sec and 68°C for 2 min;
and final extension was at 72°C for 5 min.

The PCR products of about 800 bp were purified from agarose gel using the QIAquick Gel Extraction Kit (QIAGEN), and then cloned into the pGEM-T Easy Vector (Promega). After sequencing, the plasmid was digested with PvuII (TaKaRa), and the fragment of interest was recovered. The recovered fragment was ligated to pBacPAK9-scVB22B pre-digested with PvuII (TaKaRa) to construct pBacPAK9-VB22B sc(Fv)2. After the resulting vector was digested with EcoRI and NotI (both from TaKaRa), the fragment of about 1,800 bp was purified from agarose gel using the QIAquick Gel Extraction Kit (QIAGEN). The fragment was then cloned into a pCXND3 expression vector to construct pCXND3-VB22B sc(Fv)2.

### 2.4 Expression of single-chain anti-human Mpl antibody in animal cells

A cell line stably expressing the single-chain antibody was prepared from CHO-DG44 cells as described below. Gene transfer was achieved by electroporation using a Gene Pulser II (Bio-Rad). An expression vector (25 µg) and 0.75 mL of CHO-DG44 cells suspended in PBS (1 x 10⁷ cells/mL) were mixed. The resulting mixture was cooled on ice for 10 min, transferred into a cuvette, and pulsed at 1.5-kV and 25 µFD. After a ten-minute restoration period at room temperature, the electroporated cells were plated in CHO-S-SFMII medium (Invitrogen) containing 500 µg/mL Geneticin (Invitrogen). CHO cell lines expressing the single-chain antibody were established through selection. A cell line stably expressing VB22B sc(Fv)2 and its culture supernatants were obtained by this method.

The transient expression of the single-chain antibody was achieved using COS7 cells as described below. An expression vector (10 µg) and 0.75 mL of COS7 cells suspended in PBS (1 x 10⁷ cells/mL) were mixed. The resulting mixture was cooled on ice for 10 min, transferred into a cuvette, and then pulsed at 1.5-kV and 25 µFD. After a ten-minute restoration period at room temperature, the electroporated cells were plated in DMEM/10% FBS medium (Invitrogen). The cells were incubated overnight and then washed with PBS. CHO-S-SFMII medium was added and the cells were cultured for about three days. The culture supernatants for preparing the VB22B diabody were thus prepared.

### 2.5 Quantitation of single-chain anti-human Mpl antibodies in culture supernatants

The culture supernatant concentration of the single-chain anti-human Mpl antibody transiently expressed in COS7 cells or CHO cells was determined using surface plasmon resonance. A sensor chip CM5 (Biacore) was placed in BIAcore 2000 (Biacore). ANTI-FLAG® M2 Monoclonal Antibody (Sigma-Aldrich) was immobilized onto the chip. An appropriate concentration of sample was injected over the chip surface at a flow rate of 5 mL/sec, and 50 mM diethylamine was used to dissociate the bound antibody. Changes in the mass during sample injection were recorded, and the sample concentration was calculated from the calibration curve prepared using the mass changes of a standard sample. db12E10 (see Japanese Patent Application No. 2001-27734) was used as the diabody standard, and 12E10 sc(Fv)2 which has the same gene structure as that of sc(Fv)2 was used as the sc(Fv)2 standard.

### 2.6 Purification of anti-Mpl diabodies and single-chain antibodies

The culture supernatants of VB22B diabody-expressing COS7 cells or CHO cells was loaded onto an Anti-Flag M2 Affinity Gel (Sigma-Aldrich) column equilibrated with a 50 mM Tris-HCl buffer (pH7.4) containing 150 mM NaCl and 0.05% Tween 20. The absorbed antibodies were eluted with 100 mM glycine-HCl (pH3.5). The fractions eluted were immediately neutralized with 1 M Tris-HCl (pH8.0), and loaded onto a HiLoad 26/60 Superdex 200 pg (Amersham Biosciences) column for gel filtration chromatography. PBS/0.01% Tween 20 was used in the gel filtration chromatography.

VB22B sc(Fv)2 was purified from the culture supernatants of VB22B sc(Fv)2-expressing COS7 cells or CHO cells under the same conditions used for purifying the diabodies. A large-scale preparation of VB22B sc(Fv)2 was prepared by loading the CHO cell culture supernatants onto a Macro-Prep Ceramic Hydroxyapatite Type I (Bio-Rad) column equilibrated with a 20 mM phosphate buffer (pH6.8), and eluting the VB22B sc(Fv)2 in a stepwise manner with 250 mM phosphate buffer (pH6.8). The eluted fraction was concentrated on an ultrafilter, and then fractionated by gel filtration chromatography using a HiLoad 26/60 Superdex 200 pg (Amersham Biosciences) column, and a fraction corresponding to the molecular weight range of about 40 kD to 70 kD was obtained. The fraction was loaded onto an Anti-Flag M2 Affinity Gel column equilibrated with a 50 mM Tris-HCl buffer (pH7.4) containing 150 mM NaCl and 0.05% Tween 20. The absorbed antibody was eluted with 100 mM glycine-HCl (pH3.5). The eluted fraction was immediately neutralized with 1 M Tris-HCl (pH8.0), and loaded onto a HiLoad 26/60 Superdex 200 pg (Amersham Biosciences) column for gel filtration chromatography. 20 mM acetate (pH6.0) containing 150 mM NaCl and 0.01 % Tween 80 was used in the gel filtration chromatography.

In each purification step, the presence of the diabody and sc(Fv)2 in the samples was confirmed by SDS-PAGE and Western blotting using an anti-Flag antibody (Sigma-Aldrich). Specifically, obtained fractions corresponding to each peak were subjected to the electrophoresis according to the method described by Laemli, and then stained using Coomassie Brilliant Blue. As a result, single band was detected apparently at about 29 kDa for the diabody; while single band was detected apparently at about 55 kDa for sc(Fv)2.

### 2.7 Assessment of TPO-like agonist activity for single-chain anti-human Mpl antibodies

TPO-like agonist activity was assessed using BaF3-human Mpl that proliferate in a TPO-dependent manner. The cells were washed twice with RPMI 1640/1% FBS (fetal bovine serum) (Invitrogen), and then suspended in RPMI 1640/10% FBS to a concentration of 4x 10⁵ cells/mL. Cell suspensions were aliquoted at 60 µL/well into a 96-well plate. Various concentrations of rhTPO (R&D) and COS7 culture supernatants or purified samples were prepared, and a 40 µL aliquot was added into each well. The plates were then incubated at 37°C under 5% CO₂ for 24 hr. Immediately after a 10-µL aliquot of WST-8 reagent (Cell Count Reagent SF; Nacalai Tesque) was added into each well, absorbance was measured at 450 nm (and at 655 nm as a control) using Benchmark Plus. After two hours of incubation, absorbance was again measured at 450 nm (and at 655 nm as a control). The WST-8 reagent changes colors at 450 nm in a color reaction that reflects the viable cell count. The TPO-like agonist activity was assessed using the change in absorbance during the two-hour incubation as an index. EC₅₀ values were computed using GraphPad Prism.

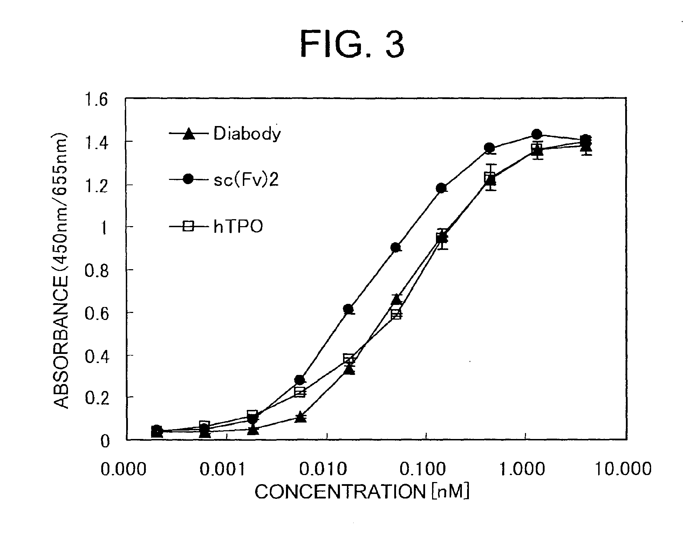

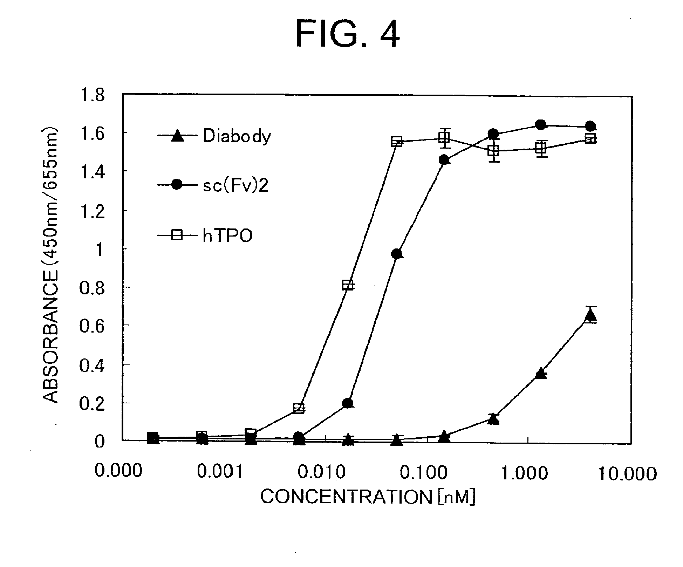

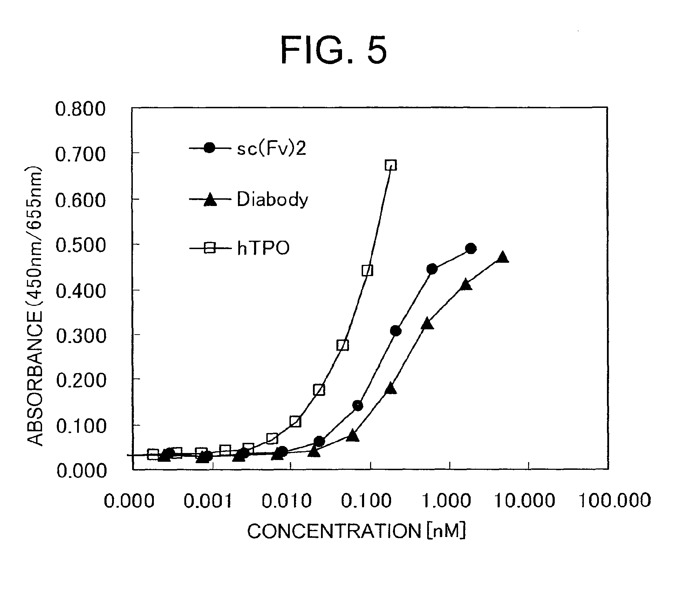

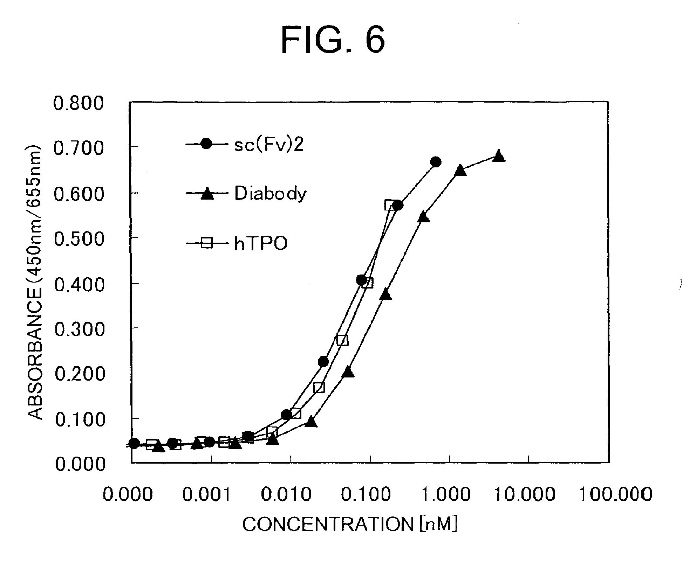

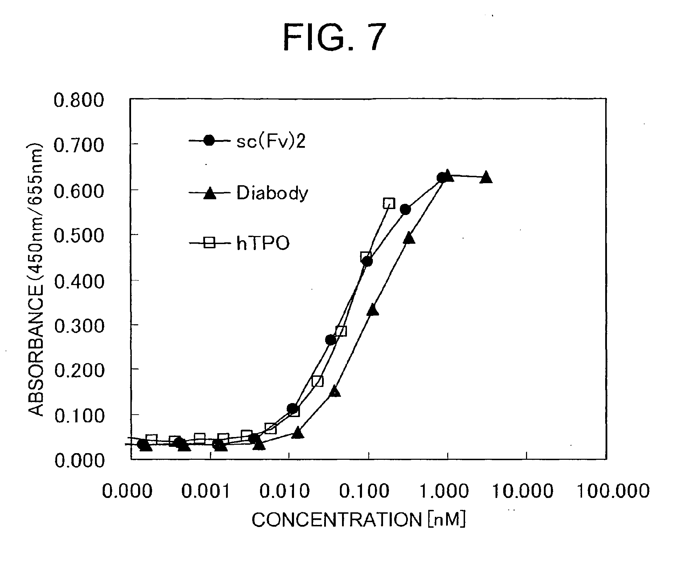

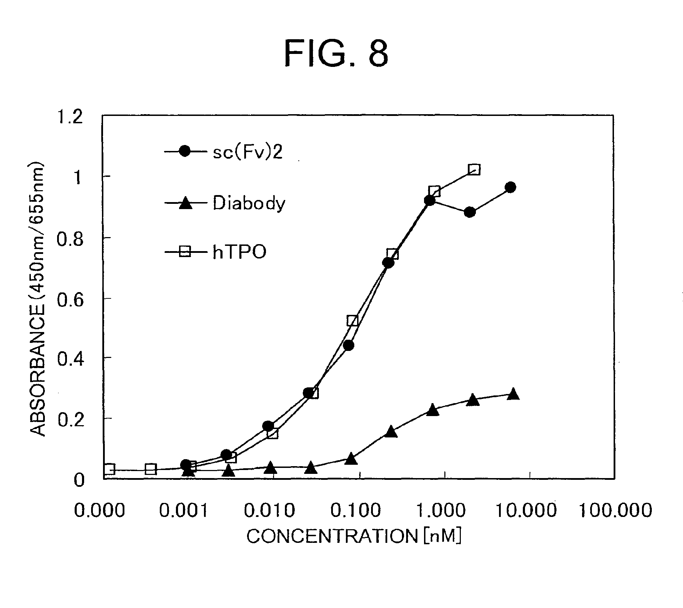

The results of assessing the TPO-like agonist activity of the purified VB22B diabody and VB22B sc(Fv)2 are shown in Fig. 3 (BaF3-human Mpl) and Fig. 4 (BaF3-monkey Mpl). Furthermore, the single-chain antibodies (diabody and sc(Fv)2) of VB16 (Fig. 5), VB 140 (Fig. 6), VB45B (Fig. 7), and TA136 (Fig. 8) are expressed in COS7 cells and the culture supernatants were used to assess the antibodies' TPO-like agonist activity in BaF3-human Mpl. In addition, the EC₅₀ values obtained from the analyses described above are shown in Table 1.

**Table 1**

| The agonistic activities (EC₅₀ value: pM) of antibodies VB22B, VB16, VB 140, VB45B, and TA136 in BaF3-human Mpl and BaF3-monkey Mpl. | | | | |
|---|---|---|---|---|
| Antibody | BaF-human Mpl | | BaF-monkey Mpl | |
| | Diabody | sc(Fv)2 | Diabody | sc(Fv)2 |
| VB22B | 61 | 27 | 1668 | 26 |
| VB16 | 190 | 95 | | |
| VB140 | 89 | 38 | | |
| VB45B | 76 | 30 | | |
| TA136 | 3076 | 54 | | |

These results confirmed that the sc(Fv)2 single-chain antibodies exhibit a higher agonistic activity than the diabodies. For the agonistic activity, it is essential that the antigen binding site is divalent. The distance and angle between two antigen binding sites are also considered to be important factors (see WO 02/33072 and WO 02/33073). The optimal distance and angle vary depending on the epitope recognized by each of the antibodies obtained. The optimal linker length differs from antibody to antibody. However, it has been reported that when the linker length is as short as 5-12mer, a non-covalent diabody is formed; and when the linker length is longer (12mer or longer), a scFv monomer is formed instead of a diabody (Hudson et al., J Immunol. Methods 1999, 231:177-189). Thus, it can be predicted that sc(Fv)2, in which a divalent antigen-binding site is formed regardless of the use of a long linker, is very likely to have a high agonistic activity. In addition, since the sc(Fv)2 which is linked by a linker is more stable, there is a possibility that it can confer a higher activity as compared with a non-covalent diabody.

### Industrial Applicability

According to the present invention, even if a full-size antibody exhibits low or no agonistic activity, the activity of the antibody can be enhanced by reducing its molecular weight, specifically, by converting the antibody to a diabody or sc(Fv)2. Thus, full-size antibodies, whose development into pharmaceuticals has been conventionally difficult due to their low activities, can be developed into pharmaceuticals if converted to minibodies. In addition, the manufacturing cost can be reduced if specific activity is improved. Furthermore, since minibodies do not bind sugar chains, various expression systems, such as animal cells, yeast, and *E. coli,* can be easily utilized for the preparation of recombinant minibodies.

## Claims

1. A method for enhancing the activity of an antibody, which comprises making the antibody into a single-chain polypeptide comprising two or more light chain variable regions and two or more heavy chain variable regions linked via linkers.

2. A method for enhancing the activity of an antibody, which comprises linking a first polypeptide to a second polypeptide by a linker, wherein the first polypeptide comprises the antibody's heavy chain variable region and light chain variable region and the second polypeptide comprises the antibody's heavy chain variable region and light chain variable region.

3. A method for enhancing the activity of an antibody, which comprises converting the antibody into an sc(Fv)2.

4. The method according to any one of claims 1 to 3, wherein the activity is an agonistic activity.

5. The method according to any one of claims 1 to 4, wherein the linker is a peptide linker.

6. The method according to claim 5, wherein the length of the peptide linker is 5 to 30 amino acids.

7. The method according to claim 6, wherein the length of the peptide linker is 12 to 18 amino acids.

8. The method according to claim 7, wherein the length of the peptide linker is 15 amino acids.

9. An antibody whose activity has been enhanced by the method according to any one of claims 1 to 8.

10. A method for producing the antibody of claim 9, which comprises:
(a) preparing a DNA that encodes two or more antibody heavy chain variable regions, two or more antibody light chain variable regions, and peptide linkers linking each of the variable regions;
(b) constructing a vector comprising the DNA;
(c) introducing the vector into a host cell; and
(d) culturing the host cell.

11. The production method according to claim 10, wherein the DNA encodes two heavy chain variable regions, two light chain variable regions, and three peptide linkers.

12. The production method according to claim 11, wherein the DNA is encoded in the order of: heavy chain variable region, peptide linker, light chain variable region, peptide linker, heavy chain variable region, peptide linker, and light chain variable region.
